# EUROPÄISCHE PATENTSCHRIFT

(11) **EP 3 501 591 B1**
(45) Veröffentlichungstag und Bekanntmachung des Hinweises auf die Patenterteilung: **24.08.2022**
(21) Anmeldenummer: 18213369.4
(22) Anmeldetag: 18.12.2018
(51) Int. Cl.: A61M 35/00, B65D 47/42, A61F 13/40, B65D 47/44, A61F 13/84

(54) **MEDIZINISCHER APPLIKATOR ZUM AUFTRAGEN EINER FLÜSSIGKEIT, INSBESONDERE EINES ANTISEPTIKUMS, AUF EINEN TUPFER**
MEDICAL APPLICATOR FOR APPLYING A LIQUID, IN PARTICULAR AN ANTISEPTIC TO A SWAB
APPLICATEUR MÉDICAL PERMETTANT D'APPLIQUER UN LIQUIDE, EN PARTICULIER UN ANTISEPTIQUE, SUR UN TAMPON

(30) Priorität: 19.12.2017 DE 102017130529
(43) Veröffentlichungstag der Anmeldung: 26.06.2019
(73) Patentinhaber: Bode Chemie GmbH, 22525 Hamburg (DE)
(72) Erfinder: LANGLOTZ, Christian, 20253 Hamburg (DE)
(74) Vertreter: Jäger, Christoph

(56) Entgegenhaltungen:
- CN-A- 105 999 457
- US-A1- 2009 259 204
- US-A1- 2013 108 352

## Beschreibung

Die vorliegende Erfindung betrifft einen medizinischen Applikator zum Auftragen einer Flüssigkeit, insbesondere eines Antiseptikums, auf einen Tupfer. Weiterhin betrifft die vorliegende Erfindung einen Kit umfassend den vorgenannten Applikator und ein Verfahren zum Auftragen einer Flüssigkeit, insbesondere eines Antiseptikums, auf einen Tupfer. In dem Verfahren wird der vorgenannte Applikator eingesetzt.

Vor einem chirurgischen Eingriff wird die Hautoberfläche des Patienten an der Operationsstelle üblicherweise mit einer antiseptischen Flüssigkeit behandelt. Dadurch werden die auf der Hautoberfläche befindlichen Keime abgetötet und das Infektionsrisiko während des chirurgischen Eingriffes minimiert. Es sind verschiedene Möglichkeiten bekannt, wie die Hautoberfläche des Patienten mit der antiseptischen Flüssigkeit behandelt werden kann.

Eine Möglichkeit zur Behandlung der Hautoberfläche mit der antiseptischen Flüssigkeit besteht darin, die antiseptische Flüssigkeit in einer Schale vorzulegen und einen Tupfer in Verbindung mit einer Pinzette oder einer Tupferzange als Greifwerkzeug zu verwenden. Der Tupfer wird in die Schale eingebracht und mit der antiseptischen Flüssigkeit getränkt. Die Hautoberfläche wird dann mit dem feuchten Tupfer abgewischt. Mit der Pinzette beziehungsweise der Zange kann der Tupfer währenddessen gehalten werden. Damit wird ein direkter Kontakt von Patient und medizinischen Personal während der Hautdesinfektion verhindert und das Infektionsrisiko weiter reduziert. Nachteilig an der beschriebenen Vorgehensweise ist, dass die antiseptische Flüssigkeit in der Schale vorgelegt werden muss. Dies ist ein zusätzlicher Arbeitsschritt für das medizinische Personal, da die antiseptische Flüssigkeit aus einem Vorratsbehälter in die Schale umgefüllt werden muss. Dabei wird die antiseptische Flüssigkeit in der Regel auch im Überschuss in die Schale gefüllt, wodurch viel Desinfektionsmittel verbraucht wird.

Im Stand der Technik sind auch Applikatoren bekannt, um die Hautoberfläche mit der antiseptischen Flüssigkeit zu behandeln. Ein solcher Applikator ist zum Beispiel in der Patentanmeldung US2002/0076255A1 beschrieben. Der Applikator umfasst einen Griff mit einem geschlossenen Ende und einem offenen Ende. Eine zerbrechliche Glasampulle mit der antiseptischen Flüssigkeit befindet sich in dem Griff. Weiterhin umfasst der Applikator ein Schaumstoffkissen am offenen Ende des Griffes und einen Öffnungsmechanismus für die Glasampulle. Dabei handelt es sich um einen Hebel oder Knopf, der die Glasampulle bei Betätigung zerbricht, so dass die antiseptische Flüssigkeit aus der Glasampulle fließen und aus dem Applikator über das Schaumstoffkissen austreten kann. In der Anwendung wird der Applikator an dem Griff gehalten und das Schaumstoffkissen mit der zu desinfizierenden Haut in Kontakt gebracht. Nachteilig an dem vorgenannten Applikator ist, dass das Schaumstoffkissen im Falle einer Kontamination nicht ausgetauscht werden kann. Kommt es also zu einer Kontamination des Schaumstoffkissens, muss der gesamte Applikator mitsamt gegebenenfalls noch vorhandener antiseptischer Flüssigkeit entsorgt werden. Außerdem ist der Öffnungsmechanismus nicht ausreichend gegen eine unbeabsichtigte Betätigung gesichert.

Die US 2013/0108352 A1 offenbart einen Applikator zum Ausgeben eines Klebstoffs für chirurgische Anwendungen. Der Applikator ist stiftartig aufgebaut und weist einen Griff auf. Der Griff ist im vorderen Teil mit einer Spitze aus einem porösen Material zum Ausgeben des Klebstoffs unlösbar verbunden. Auf den hinteren, zylinderförmigen Teil des Griffs kann ein zunächst noch verschlossener Behälter mit dem Klebstoff gesteckt werden, wobei eine ringartige Sicherung eine Öffnung des Behälterverschlusses verhindert. Nach Entfernen der Sicherung von dem Applikator kann der Behälter weiter auf den Griff des Applikators geschoben werden. Dabei kann das Ende des Griffs so tief in den Behälter eindringen, dass dessen Verschluss geöffnet wird und der Klebstoff aus dem Behälter durch den Griff zur Spitze fließen kann.

Die CN 105999457 A offenbart einen Applikator für ein Desinfektionsmittel, welcher in der Gynäkologie zum Einsatz kommen soll. Der Applikator umfasst eine Klemmvorrichtung für ein Reinigungsmaterial sowie auch elektronische Komponenten.

Der vorliegenden Erfindung liegt die Aufgabe zugrunde, die Hautdesinfektion mit einem Applikator für eine antiseptische Flüssigkeit zu verbessern und die Nachteile im Stand der Technik zu überwinden. Insbesondere liegt der vorliegenden Erfindung die Aufgabe zugrunde, einen Applikator für eine antiseptische Flüssigkeit zur Hautdesinfektion mit einem einfach aufgebauten und sicheren Öffnungsmechanismus zur Verfügung zu stellen. Weiterhin liegt der vorliegenden Erfindung die Aufgabe zugrunde, einen einfach anwendbaren sowie vielseitig einsetzbaren Applikator für eine antiseptische Flüssigkeit zur Hautdesinfektion bereitzustellen. Die Erfindung löst die Aufgaben mit einem medizinischen Applikator nach Anspruch 1, einem Kit nach Anspruch 16 und einem Verfahren nach Anspruch 17.

Die Erfindung betrifft demnach gemäß einem ersten Aspekt einen medizinischen Applikator zum Auftragen einer Flüssigkeit, insbesondere eines Antiseptikums, auf einen Tupfer nach Anspruch 1.

Gemäß einem zweiten Aspekt betrifft die Erfindung ein Kit nach Anspruch 16.

Ein dritter Aspekt der Erfindung ist ein Verfahren zum Auftragen einer Flüssigkeit, insbesondere eines Antiseptikums, auf einen Tupfer nach Anspruch 17.

Der erfindungsgemäße medizinische Applikator zum Auftragen einer Flüssigkeit auf einen Tupfer umfasst einen flexiblen Behälter mit der Flüssigkeit und mit einem einen Membranverschluss aufweisenden ersten Auslass. Der Behälter enthält also eine Flüssigkeit, bei der es sich typischerweise um ein Antiseptikum handelt. Der Behälter besitzt weiterhin einen Auslass, welcher im Zusammenhang mit der vorliegenden Erfindung auch als erster Auslass bezeichnet wird. Der Auslass des Behälters ist mit einem Membranverschluss versehen, womit vorliegend auch ein Septum gemeint ist. Derartige Verschlüsse sind bekannt und werden beispielsweise für Ampullen eingesetzt. Der Membranverschluss kann geöffnet werden. Wenn dies geschieht, kann die Flüssigkeit aus dem Behälter über den Auslass austreten. Der Austritt der Flüssigkeit kann dadurch bewirkt oder unterstützt werden, indem der Behälter zusammengedrückt wird. Hierfür muss der Behälter flexibel ausgebildet sein. Ein flexibler Behälter kann verformt und zumindest teilweise zusammengedrückt werden ohne dabei zu Bruch zu gehen.

Der erfindungsgemäße medizinische Applikator umfasst weiterhin ein Aufsatzteil für den Behälter. Das Aufsatzteil umfasst einen zweiten Auslass, mindestens ein Perforationselement, einen entfernbaren Abstandshalter sowie einen Greifmechanismus. Das Perforationselement hat die Funktion, den Membranverschluss zu durchstoßen und somit zu öffnen. Der Abstandshalter beabstandet den Membranverschluss von dem Perforationselement. Dadurch kann der Membranverschluss nicht von dem Perforationselement geöffnet werden, solange der Abstandshalter an dem Applikator vorhanden ist. Der Abstandshalter kann somit den intakten Zustand des Membranverschlusses sicherstellen und ein unbeabsichtigtes Öffnen des Membranverschlusses verhindern. Der Greifmechanismus wiederum dient dazu, den Tupfer zu ergreifen und zu halten.

Das Aufsatzteil ist an dem Behälter in der Art einer Kappe befestigt. Nachdem der Verschluss des Behälters wie nachfolgend beschrieben geöffnet wurde, kann die Flüssigkeit den Behälter über den ersten Auslass verlassen und durch das Aufsatzteil zu dem zweiten Auslass fließen. Von dort kann die Flüssigkeit nach außen abgegeben werden.

Wesentlich an dem erfindungsgemäßen medizinischen Applikator ist weiterhin, dass er verschiedene Anordnungen beziehungsweise Konfigurationen einnehmen kann. So kann der Applikator eine erste Anordnung und eine zweite Anordnung einnehmen. In der ersten Anordnung ist der Abstandshalter am Applikator vorhanden und der Membranverschluss folglich intakt. Typischerweise stellt der Abstandshalter den intakten Zustand des Membranverschlusses in der ersten Anordnung dadurch sicher, indem er das befestigte Aufsatzteil bereits an den Behälter anstoßen lässt, wenn der Membranverschluss und das Perforationselement noch voneinander beabstandet sind und noch nicht miteinander wechselwirken können. In der zweiten Anordnung ist der Abstandshalter am Applikator nicht vorhanden und der Membranverschluss geöffnet. Der Applikator kann von der ersten Anordnung in die zweite Anordnung überführt werden, indem der Abstandshalter von dem Applikator entfernt und das Aufsatzteil in Richtung Behälter bewegt, insbesondere geschoben, gedreht oder geschraubt, wird. Infolgedessen wird der Abstand von Membranverschluss und Perforationselement so weit verringert, dass das Perforationselement den Membranverschluss durchstoßen und öffnen kann.

Der Öffnungsmechanismus des erfindungsgemäßen medizinischen Applikators erfordert nur wenige Komponenten und lässt sich herstellerseitig leicht umsetzen. Der Öffnungsmechanismus ist also einfach aufgebaut. Gleichzeitig ist der Öffnungsmechanismus wie bereits erwähnt gegen eine unbeabsichtigte Betätigung gesichert und einfach in der Anwendung. Vorteilhaft an dem hiermit vorgeschlagenen Applikator ist ebenfalls, dass der Anwender schnell erkennen kann, ob der Applikator unbenutzt ist oder bereits verwendet wurde. So kann der Anwender einen unbenutzten Applikator an dem vorhandenen Abstandshalter erkennen. Wurde der Applikator hingegen bereits verwendet, kann der Anwender dies anhand des am Applikator fehlenden Abstandshalters erkennen.

Wie bereits eingangs erwähnt, handelt es sich bei der Flüssigkeit in dem Behälter typischerweise um eine antiseptische Flüssigkeit (Antiseptikum), insbesondere um eine sterile antiseptische Flüssigkeit. Antiseptika sind im Stand der Technik bekannt und umfassen beispielsweise Alkohole wie Ethanol, 1-Propanol und 2-Propanol, quartäre Ammoniumverbindungen wie Benzalkoniumchlorid, Octenidin, iodhaltige Verbindungen wie Povidon-lod sowie halogenierte Verbindungen wie Chlorhexidin. Vorzugsweise ist die Flüssigkeit in dem Behälter ein Antiseptikum umfassend eine quartäre Ammoniumverbindung, Octenidin oder Chlorhexidin. Besonders bevorzugt handelt es sich bei der Flüssigkeit in dem Behälter um ein Antiseptikum umfassend eine Mischung aus (a) einem Alkohol (zum Beispiel Ethanol, 1-Propanol oder 2-Propanol), und (b) einer quartären Ammoniumverbindung, Octenidin oder Chlorhexidin. Ein solches Antiseptikum tötet Keime schnell (durch die alkoholische Wirkkomponente) und lang anhaltend (durch die Remanenzwirkstoffe (b) wie Octenidin und Chlorhexidin) ab.

Der Greifmechanismus ist erfindungsgemäß manuell und zangenartig ausgestaltet und umfasst einen ersten Greifarm und einen zweiten Greifarm. Ein solcher Greifmechanismus kann mit der Hand bedient werden (daher die Bezeichnung manuell) und ähnelt in seinem prinzipiellen Aufbau und seiner prinzipiellen Wirkweise einer Zange (daher die Bezeichnung zangenartig). Der Greifmechanismus kann dann wiederholt geöffnet und geschlossen werden, so dass der Tupfer nicht nur ergriffen, sondern auch wieder freigegeben werden kann. Der manuelle und zangenartige Greifmechanismus ist intuitiv und einfach anwendbar, weil er den seit langer Zeit bekannten Tupferzangen ähnelt. Zudem ist er vielseitig sowie flexibel einsetzbar, weil er wiederholt betätigt werden kann und somit einen Tupferwechsel möglich macht. Tupferwechsel sind bei der Desinfektion eines Hautareals in der Hautantiseptik oft erforderlich und vorgeschrieben, zum Beispiel wenn das Hautareal groß ist oder wiederholt desinfiziert werden soll.

Vorzugsweise ist der Behälter im Wesentlichen zylinderförmig (kreiszylinderförmig) ausgestaltet. Die Zylinderform bezieht sich dabei auf den nicht zusammengedrückten Behälter. Ein zylinderförmiger Behälter mit einem dazu passenden Aufsatzteil kann gut in der Hand gehalten werden und schränkt die Bewegungsfreiheit der Greifarme nicht ein. Der Auslass des Behälters (erster Auslass) mit dem Membranverschluss ist typischerweise an einer Oberseite des Behälters positioniert. Die Oberseite eines zylinderförmigen Behälters entspricht geometrisch betrachtet der Deckfläche des Zylinders. Da der Behälter flexibel sein muss, um zusammengedrückt werden zu können, umfasst das den Behälter bildende Material typischerweise einen Kunststoff, insbesondere einen biegsamen Kunststoff. Vorzugsweise besteht der Behälter aus einem Kunststoff, insbesondere einem biegsamen Kunststoff, wobei der Membranverschluss des Behälters gegebenenfalls aus einem anderen Material bestehen kann.

Der Auslass des Aufsatzteils (zweiter Auslass) kann eine einzige Öffnung aufweisen. Ein Aufsatzteil mit einer einzigen Öffnung als Auslass lässt sich leicht herstellen. Alternativ kann der Auslass des Aufsatzteils auch mehrere Öffnungen umfassen. Diese mehreren Öffnungen sind typischerweise kleiner als die zuvor genannte einzige Öffnung und bilden vorzugsweise eine siebartige Anordnung aus. Damit kann die Flüssigkeit besser auf den Tupfer verteilt werden. Die Anzahl der mehreren Öffnungen kann 2 bis 20, bevorzugt 3 bis 15, mehr bevorzugt 3 bis 10 und ganz besonders bevorzugt 3 bis 6 betragen.

Üblicherweise umfasst das Aufsatzteil einen Hohlkörper mit einem ersten offenen Ende und einem zweiten offenen Ende. Der Hohlkörper wird mit seinem ersten offenen Ende an dem Behälter befestigt. Dabei nimmt der Hohlkörper den Auslass des Behälters auf. Das zweite offene Ende bildet den zweiten Auslass und ist dem ersten offenen Ende vorzugsweise gegenüberliegend angeordnet. Das Perforationselement ist in dem Hohlkörper angeordnet, während der Abstandshalter an dem ersten offenen Ende angeordnet ist. Der erste und der zweite Greifarm sind außen an dem Hohlkörper befestigt. Vorzugsweise sind der erste und der zweite Greifarm außen an dem Hohlkörper an zwei sich im Wesentlichen gegenüberliegenden Positionen befestigt. Wenn sich die Greifarme gegenüberliegen, kann eine optimale Handhabbarkeit des Greifmechanismus erzielt werden. Der Hohlkörper stellt eine Art Basis des Aufsatzteils dar, an oder in dem die weiteren Komponenten des Aufsatzteils wie beispielsweise der Abstandshalter oder das Perforationselement angeordnet sind. Wenn das Aufsatzteil wie vorangehend beschrieben aufgebaut ist, kann die Flüssigkeit in dem Behälter bei geöffnetem Verschluss von dem ersten Auslass zu dem zweiten Auslass fließen. Dabei wird die Flüssigkeit von dem Innenraum des Hohlkörpers von dem ersten Auslass zu dem zweiten Auslass geleitet.

Der Hohlkörper kann beispielsweise im Wesentlichen die Form eines Zylinders (Kreiszylinders) oder eines Kegelstumpfes aufweisen. Eine Grundfläche des Zylinders oder des Kegelstumpfes umfasst das erste offene Ende des Hohlkörpers, welches zur Befestigung des Aufsatzteils am Behälter dient. Eine Deckfläche des Zylinders oder des Kegelstumpfes umfasst das zweite offene Ende des Hohlkörpers, welches den zweiten Auslass bildet. Der erste und der zweite Greifarm sind außen an einer Mantelfläche des Zylinders oder des Kegelstumpfes befestigt. Ein Aufsatzteil mit einem solchen Hohlkörper kann mit dem zylinderförmigen Behälter gut zusammenpassen.

Das Aufsatzteil des Applikators umfasst wie bereits erwähnt vorteilhafterweise einen manuellen, zangenartigen Greifmechanismus mit einem ersten Greifarm und einem zweiten Greifarm. Dabei umfasst sowohl der erste als auch der zweite Greifarm vorzugsweise einen Kopfabschnitt, einen Gelenkabschnitt und einen Griffabschnitt.

Der Kopfabschnitt ist zum Ergreifen und zum Halten des Tupfers vorgesehen ist und kann eine Greifbacke umfassen. Entsprechend ist der Kopfabschnitt vor dem zweiten Auslass angeordnet.

Der Gelenkabschnitt ist zur beweglichen Befestigung des Greifarms am Aufsatzteil vorgesehen und ist normalerweise außen an dem Hohlkörper angeordnet. Der Gelenkabschnitt kann eine scharnierartige Verbindung umfassen, um den Greifarm beweglich an dem Aufsatzteil (beziehungsweise dessen Hohlkörper) zu befestigen. Es ist auch möglich, dass das Aufsatzteil (beziehungsweise dessen Hohlkörper) und der Greifarm einstückig ausgebildet sind, wobei die Verbindung zwischen Aufsatzteil und Greifarm flexibel ist und den Gelenkabschnitt ausbildet.

Der Griffabschnitt ist zum Halten und zur Betätigung des Greifmechanismus vorgesehen. Der Griffabschnitt ist behälterseitig angeordnet und weist somit in Richtung des Behälters. Wenn beide Greifarme einen Kopfabschnitt, einen Gelenkabschnitt und einen Griffabschnitt umfassen, kann ein sehr beweglicher und leicht handhabbarer Greifmechanismus erhalten werden.

Alternativ kann es jedoch auch vorteilhaft sein, die Greifarme unterschiedlich auszugestalten. So kann der erste Greifarm einen Kopfabschnitt umfassen und feststehend mit dem Aufsatzteil verbunden sein, wobei der erste Greifarm an der Verbindungsstelle mit dem Aufsatzteil endet. Der zweite Greifarm umfasst dann einen Kopfabschnitt, einen Gelenkabschnitt und einen Griffabschnitt. Auch in dieser alternativen Ausführungsform können die Kopfabschnitte der beiden Greifarme jeweils eine Greifbacke umfassen, um den Tupfer besser ergreifen zu können. Zudem sind die Verbindungsstelle des ersten Greifarms mit dem Aufsatzteil und der Gelenkabschnitt des zweiten Greifarms normalerweise außen an dem Hohlkörper angeordnet. Wenn wie in dieser alternativen Ausführungsform vorgeschlagen ein Greifarm feststehend ausgebildet wird, kann ein einfacher und leicht herstellbarer Greifmechanismus erhalten werden, weil der feststehende Greifarm keinen Gelenkabschnitt und keinen Griffabschnitt umfassen muss.

Weiterhin ist der Greifmechanismus vorzugsweise derart ausgestaltet, dass er in einem unbetätigten Zustand im Wesentlichen geschlossen oder vollständig geschlossen ist und bei Betätigung geöffnet werden kann. Ein solcher Greifmechanismus kann den Tupfer im unbetätigten Zustand selbstständig fixiert halten, womit die Handhabbarkeit des Applikators weiter vereinfacht wird. Insbesondere gestattet ein solcher Greifmechanismus die Benutzung des Applikators mit nur einer Hand, um den Tupfer zu ergreifen und anschließend die Flüssigkeit auf den Tupfer aufzutragen.

Anknüpfend an die vorangegangene bevorzugte Ausführungsform kann das Aufsatzteil mindestens ein federndes Element umfassen, so dass sich der Greifmechanismus selbstständig in eine Ausgangsstellung bewegen kann, wenn sich der Greifmechanismus in einem unbetätigten Zustand befindet. Der erste und der zweite Greifarm, insbesondere der Kopfabschnitt des ersten und des zweiten Greifarms, sind dann derart ausgestaltet, dass der Greifmechanismus in der Ausgangsstellung im Wesentlichen geschlossen oder vollständig geschlossen ist und bei Betätigung geöffnet werden kann. Beispielsweise können die Kopfabschnitte entgegengesetzt bogenförmig ausgestaltet sein und in der Ausgangsstellung eine ovalförmige Anordnung bilden, damit der Greifmechanismus in der Ausgangsstellung im Wesentlichen geschlossen oder vollständig geschlossen ist und bei Betätigung geöffnet werden kann.

Ebenso kann der Greifmechanismus aber auch derart ausgestaltet sein, dass er in einem unbetätigten Zustand geöffnet ist und bei Betätigung geschlossen werden kann. Ein solcher Greifmechanismus hat den Vorteil, dass der Anwender des Applikators selbst bestimmen kann, mit welcher Kraft der Tupfer fixiert wird. Insbesondere kann der Tupfer mit einem solchen Greifmechanismus mit hoher Kraft fixiert werden, um ein unbeabsichtigtes Lösen des Tupfers von dem Applikator zu verhindern. Die Zuverlässigkeit des Applikators wird dadurch erhöht.

In Weiterführung der zuletzt beschriebenen Ausführungsform kann das Aufsatzteil mindestens ein federndes Element umfassen, so dass sich der Greifmechanismus selbstständig in eine Ausgangsstellung bewegen kann, wenn sich der Greifmechanismus in einem unbetätigten Zustand befindet. Der erste und der zweite Greifarm, insbesondere der Kopfabschnitt des ersten und des zweiten Greifarms, sind dann derart ausgestaltet, dass der Greifmechanismus in der Ausgangsstellung geöffnet ist und bei Betätigung geschlossen werden kann. Zum Beispiel können sich die Kopfabschnitte überkreuzen und in der Ausgangsstellung eine x-förmige Anordnung bilden, damit der Greifmechanismus in der Ausgangsstellung geöffnet ist und bei Betätigung geschlossen werden kann.

In einer bevorzugten Ausführungsform der Erfindung ist der Applikator entlang einer Längsachse im Wesentlichen symmetrisch aufgebaut. Der Tupfer ist mit dem Greifmechanismus auf der Längsachse positionierbar. Damit kann der Applikator gut in der Hand gehalten und die Flüssigkeit sehr exakt auf den Tupfer aufgetragen werden.

Für den erfindungsgemäßen Applikator ist es auch vorgesehen, dass der Behälter oder das Aufsatzteil ein Flatterventil umfassen kann. Flatterventile als solche sind bekannt, zum Beispiel aus der DE19937549B4. Das Flatterventil ist in dem Behälter oder in dem Aufsatzteil angeordnet. Ein im Aufsatzteil vorhandenes Flatterventil ist normalerweise in dessen Hohlkörper angeordnet. Bei einem Applikator mit Flatterventil kann die Flüssigkeit von dem ersten Auslass zu dem zweiten Auslass erst dann fließen, wenn zusätzlich zu dem Behälterverschluss auch das Flatterventil geöffnet ist. Das Flatterventil kann geöffnet werden, indem der Behälter zusammengedrückt wird. Mit dem Flatterventil kann ein vorzeitiges Austreten der Flüssigkeit aus dem Applikator beispielsweise beim Ergreifen des Tupfers vermieden werden. Das Auftragen der Flüssigkeit auf den Tupfer ist bei einem Applikator mit Flatterventil für den Anwender besser kontrollierbar und steuerbar.

Erfindungsgemäß ist das Aufsatzteil an dem Behälter befestigt. Dies trifft auch auf den Applikator in der ersten Anordnung, also mit Abstandshalter, zu. Das Befestigen des Aufsatzteils an dem Behälter kann erfolgen, indem das Aufsatzteil dem Behälter aufgeschoben oder aufgesteckt, vorzugsweise einrastend aufgesteckt, wird. Aufsatzteil und Behälter können in diesem Fall also mittels einer steckerartigen Verbindung oder mittels einer Schnappverbindung aneinander befestigt sein. Um eine besonders sichere und dichte Befestigung des Aufsatzteils an dem Behälter zu erzielen, kann das Aufsatzteil an dem Behälter auch angeschraubt werden. Aufsatzteil und Behälter können hierbei folglich mittels einer schraubartigen Verbindung aneinander befestigt sein. Die Art der Verbindung hat einen Einfluss darauf, wie die Bewegung des Aufsatzteils in Richtung des Behälters erfolgt, um den Applikator von der ersten in die zweite Anordnung zu überführen. Bei der steckerartigen Verbindung wird in der Regel geschoben, während bei der schraubartigen Verbindung in der Regel gedreht beziehungsweise geschraubt wird.

Das erfindungsgemäß vorhandene Perforationselement ist in dem Aufsatzteil, insbesondere in dessen Hohlkörper, angeordnet. Bei dem Perforationselement handelt es sich bevorzugt um einen Dorn. Ein Dorn umfasst eine Spitze und kann den Membranverschluss leicht durchstoßen. Zum Beispiel kann das Aufsatzteil einen einzigen rohrartig ausgestalteten Dorn besitzen, wobei der Dorn zentral in dem Aufsatzteil angeordnet ist. Besonders vorteilhaft ist jedoch eine Ausführungsform, in der das Aufsatzteil einen einzigen oder zwei flächig ausgestaltete Dorne besitzt, wobei die Dorne so in dem Aufsatzteil angeordnet sind, dass der Membranverschluss in einem peripheren Bereich durchstoßen werden kann. Ein Aufsatzteil mit einem solchen Dorn kann die Flüssigkeit ungehindert nach außen leiten und den Membranverschluss in der Art eines Messers vollflächig aufschneiden, wenn das Aufsatzteil auf den Behälter gedreht wird.

In einer besonders bevorzugten Ausführungsform der Erfindung ist der Abstandshalter ein ringartiges Abreißelement, insbesondere ein Abreißring. Ein derartiges Abreißelement lässt sich herstellerseitig leicht an dem Aufsatzteil anbringen und realisieren. Ein solches Abreißelement lässt sich auch leicht wieder von dem Aufsatzteil manuell entfernen, wenn der Applikator von der ersten Anordnung in die zweite Anordnung überführt werden soll. Denkbar ist weiterhin, dass der Abstandshalter in der ersten Anordnung nicht nur mit dem Aufsatzteil, sondern auch mit dem Behälter lösbar verbunden ist. Der Abstandshalter könnte dann in der ersten Anordnung verhindern, dass sich das Aufsatzteil unbeabsichtigt von dem Behälter löst und hierdurch den Zusammenhalt des Applikators verbessern.

In der Regel wird der Applikator als Einwegartikel verwendet. Der Applikator wird also nach der Verwendung nicht wieder aufbereitet, sondern entsorgt. Allgemein können Einwegartikel preisgünstiger hergestellt werden, da sie nur für kurze Zeit verwendet werden und somit geringere Anforderungen hinsichtlich ihrer Haltbarkeit bestehen. Für die Herstellung von Behälter und Aufsatzteil geeignete Kunststoffe sind zum Beispiel Polyethylen (PE), Polyethylenterephthalat (PET) und Polypropylen (PP).

Die Erfindung betrifft gemäß dem zweiten Aspekt einen Kit. Der Kit umfasst einen sterilen medizinischen Applikator nach dem ersten Aspekt der Erfindung. Dabei sind alle Komponenten des Applikators steril, also zum Beispiel auch die Flüssigkeit in dem Behälter. Optional umfasst der Kit einen oder mehrere sterile Tupfer. Optional umfasst der Kit zudem eine Gebrauchsanweisung. Mit dem Kit können dem Anwender alle Komponenten zur Verfügung gestellt werden, die er für einen Desinfektionsvorgang benötigt.

Vorzugsweise ist die Flüssigkeit in dem Behälter des Kits ein Antiseptikum umfassend eine quartäre Ammoniumverbindung, Octenidin oder Chlorhexidin. Dabei umfasst der Tupfer vorteilhafterweise synthetische Fasern, beispielsweise aus Polyethylenterephthalat (PET) oder Polypropylen (PP), weil diese die Freigabe der zuvor genannten bevorzugten Antiseptika aus dem Tupfer im Gegensatz zu Baumwollfasern nicht beeinträchtigen. Der synthetische Tupfer kann auch einen Farbstoff umfassen, um die antiseptische Flüssigkeit anzufärben, so dass der mit dem feuchten Tupfer in Kontakt gebrachte Bereich visuell leicht identifiziert werden kann. Besonders bevorzugt umfasst das Antiseptikum in dem Kit zusätzlich zu der quartären Ammoniumverbindung, Octenidin oder Chlorhexidin noch einen Alkohol als antiseptische Wirkkomponente.

Der dritte Aspekt der Erfindung betrifft ein Verfahren zum Auftragen einer Flüssigkeit, insbesondere eines Antiseptikums, auf einen Tupfer.

Das Verfahren umfasst die folgenden Schritte:
i. Bereitstellen eines medizinischen Applikators nach dem ersten Aspekt der Erfindung,
ii. Öffnen des Membranverschlusses des Behälters, indem der Abstandshalter von dem Applikator entfernt und das Aufsatzteil in Richtung Behälter bewegt, insbesondere geschoben, gedreht oder geschraubt, wird,
iii. Bereitstellen des Tupfers,
iv. Ergreifen und Halten des Tupfers mit dem Greifmechanismus,
v. optional Ausrichten des Applikators mit dem Tupfer in eine Position, in welcher die Flüssigkeit der Schwerkraft folgend aus dem Behälter auf den Tupfer aufgetragen werden kann,
vi. Auftragen eines Teils der in dem Behälter enthaltenen Flüssigkeit oder der gesamten in dem Behälter enthaltenen Flüssigkeit auf den Tupfer, indem der Behälter einmal oder mehrmals zusammengedrückt wird.

Die Bereitstellung des Applikators in Schritt i. und die Bereitstellung des Tupfers in Schritt iii. kann mit einem Kit nach dem zweiten Aspekt der Erfindung erfolgen.

Die Reihenfolge der Verfahrensschritte kann auch von dem oben angegebenen Ablauf abweichen. Zum Beispiel kann der Tupfer auch bereitgestellt und ergriffen werden, bevor der Membranverschluss geöffnet wird.

Wenn in Schritt vi. des Verfahrens nur ein Teil der Flüssigkeit auf den Tupfer aufgetragen wird, kann das Verfahren die folgenden weiteren Schritte umfassen:
i. Freigeben des Tupfers,
ii. Bereitstellen eines weiteren Tupfers,
iii. Wiederholen der Schritte iv. bis vi. des Verfahrens mit dem weiteren Tupfer.

Die Merkmale der bevorzugten Ausführungsformen des ersten Aspektes der Erfindung lassen sich entsprechend auch auf den zweiten und dritten Aspekt der Erfindung übertragen.

### Figurenbeschreibung

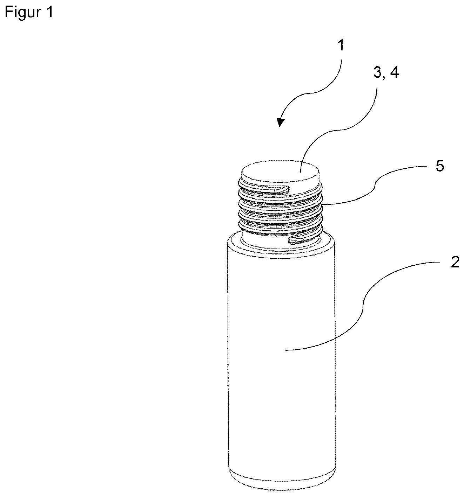

Der mit dem vorliegenden Dokument vorgeschlagene medizinische Applikator wird im Folgenden anhand der Figuren 1 bis 8 beispielhaft veranschaulicht. Dabei können identische Strukturelemente mit denselben Bezugszeichen ausgewiesen sein.

**Figur 1** zeigt einen Behälter **1** des Applikators in einer Ausführungsform. Der Behälter **1** ist mit einer Flüssigkeit **2** (nicht dargestellt), typischerweise einem Antiseptikum, gefüllt und im Wesentlichen zylinderförmig ausgestaltet. An einer Oberseite des Behälters **1** ist ein Membranverschluss **3** angeordnet, welcher einen Auslass **4** des Behälters **1** überspannt und verschließt. Im oberen Bereich des Behälters **1** befindet sich ein Außengewinde **5,** damit ein Aufsatzteil (nicht dargestellt) angeschraubt werden kann. Der Behälter **1** besteht vorzugsweise aus einem biegsamen Kunststoffmaterial, damit er wie erfindungsgemäß gefordert flexibel ist, um zusammengedrückt werden zu können. Das Erscheinungsbild des Behälters **1** ähnelt einer Ampulle.

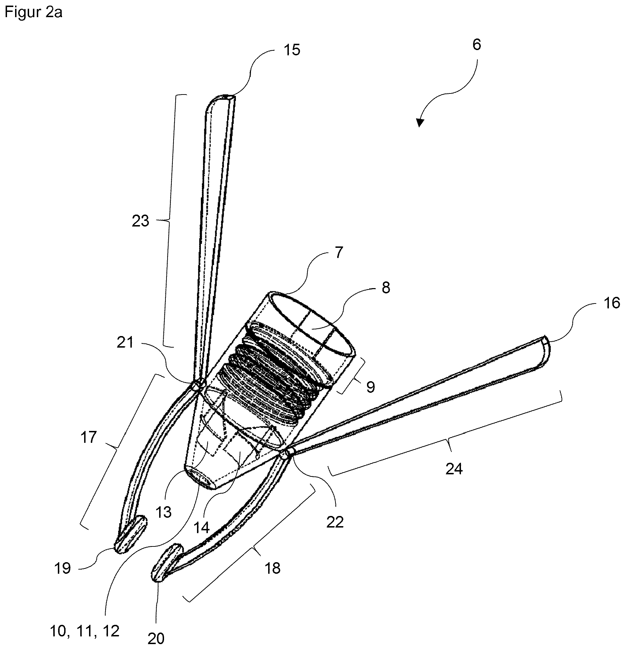

**Figur 2a** zeigt ein Aufsatzteil **6** des Applikators in einer ersten Ausführungsform. Das Aufsatzteil **6** ist passend zu dem Behälter **1** aus **Figur 1** ausgestaltet. Ein Hohlkörper **7** bildet eine Art Basis des Aufsatzteils **6** aus. Der Hohlkörper **7** hat einen zylinderförmigen Abschnitt, welcher in einen kegelstumpfförmigen Abschnitt übergeht. Außen an dem Hohlkörper **7** und auch innerhalb des Hohlkörpers **7** befinden sich weitere Komponenten des Aufsatzteils **6.** Der Hohlkörper **7** weist ein erstes offenes Ende **8** auf, an dem ein Abreißring **9** angeordnet ist. Mit einem nahe des ersten offenen Endes **8** vorhandenen Innengewinde kann das Aufsatzteil **6** an der Oberseite des Behälters **1** angeschraubt und somit befestigt werden. Dabei nimmt das erste offene Ende **8** auch den Auslass **4** des Behälters **1** mit dem Membranverschluss **3** auf. Der Hohlkörper **7** weist zudem ein zweites offenes Ende **10** auf, welches dem ersten offenen Ende **8** gegenüberliegt. Das zweite offene Ende **10** bildet einen Auslass **11** des Aufsatzteils **6** aus. Über den Auslass **11** kann Flüssigkeit nach außen abgegeben werden, wenn das Aufsatzteil **6** mit dem Behälter **1** verbunden ist. Bei dem in **Figur 2a** gezeigten Aufsatzteil **6** besteht der Auslass **11** aus einer einzigen Öffnung **12.** Des Weiteren sind in dem Hohlkörper **7** zwei flächig ausgestaltete Dorne **13, 14** zum Öffnen des Membranverschlusses **3** enthalten.

An der Mantelfläche des Hohlkörpers **7** sind ein erster Greifarm **15** und ein zweiter Greifarm **16** beweglich befestigt. Die beiden Greifarme **15, 16** bilden einen manuellen, zangenartigen Greifmechanismus, mit dem ein Tupfer ergriffen und gehalten werden kann. Sowohl der erste als auch der zweite Greifarm **15, 16** umfasst einen Kopfabschnitt **17, 18** mit einer Greifbacke **19, 20.** Mit den Kopfabschnitten **17, 18** und insbesondere deren Greifbacken **19, 20** wird der Tupfer ergriffen und gehalten. Die Kopfabschnitte **17, 18** der Greifarme **15, 16** sind entgegengesetzt bogenförmig ausgestaltet. Die Greifarme **15, 16** umfassen weiterhin jeweils einen Gelenkabschnitt **21, 22,** welche die Greifarme **15, 16** beweglich an der Mantelfläche des Hohlkörpers **7** befestigen. Die Gelenkabschnitte **21, 22** können scharnierartig ausgebildet sein. Außerdem umfassen die Greifarme **15, 16** jeweils noch einen Griffabschnitt **23, 24** zum Betätigen des Greifmechanismus. Die Griffabschnitte **23, 24** weisen einen geraden Verlauf auf und stehen dem Hohlkörper **7** in einem Winkel von in etwa 45° ab, wenn der Greifmechanismus geschlossen ist.

Der Greifmechanismus ist in **Figur 2a** in einem im Wesentlichen geschlossenen Zustand dargestellt. Vorzugsweise ist das Aufsatzteil **6** so ausgestaltet, dass die dargestellte Position der Greifarme **15, 16** in einem unbetätigten Zustand des Greifmechanismus automatisch vorliegt und gehalten wird. Dies kann zum Beispiel durch federnde Elemente (nicht dargestellt) in den Gelenkabschnitten **21, 22** erreicht werden. Die federnden Elemente drücken die Griffabschnitte **23, 24** von dem Hohlkörper **7** weg, damit der Greifmechanismus im unbetätigten Zustand geschlossen ist.

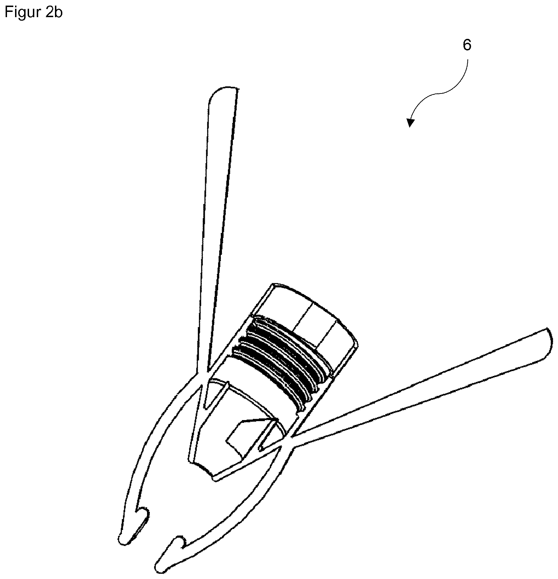

**Figur 2b** zeigt das Aufsatzteil **6** aus **Figur 2a** in einer Schnittansicht.

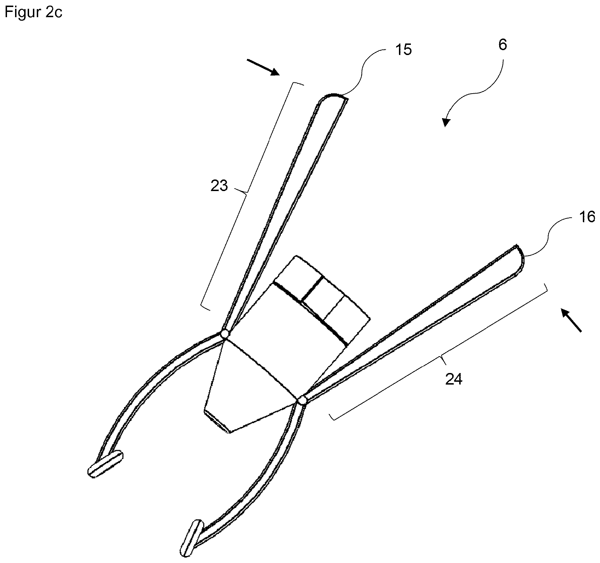

**Figur 2c** zeigt das Aufsatzteil **6** aus **Figur 2a** mit geöffnetem Greifmechanismus. Der Greifmechanismus wird geöffnet, indem die Greifarme **15, 16** an den Griffabschnitten **23, 24** in Pfeilrichtung bewegt werden.

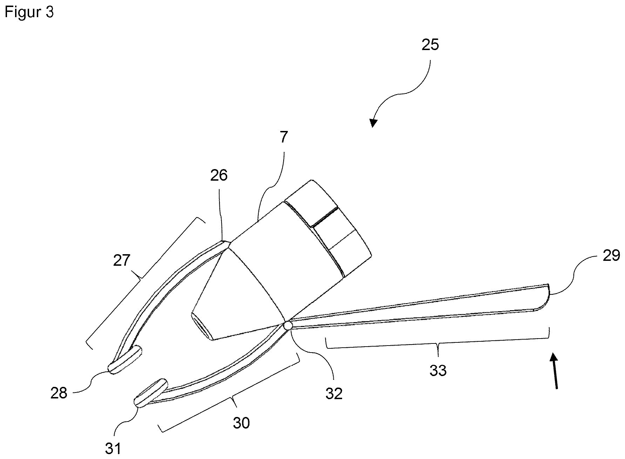

**Figur 3** zeigt ein Aufsatzteil **25** des Applikators in einer zweiten Ausführungsform. Das Aufsatzteil **25** ist sehr ähnlich wie das zuvor beschriebene Aufsatzteil **6** aufgebaut. Das Aufsatzteil **25** unterscheidet sich von dem Aufsatzteil **6** lediglich im Hinblick auf den Greifmechanismus. Der erste Greifarm **26** weist einen Kopfabschnitt **27** mit einer Greifbacke **28** auf. Der Kopfabschnitt **27** ist feststehend mit dem Hohlkörper **7** verbunden. Der zweite Greifarm **29** weist wie die Greifarme des Aufsatzteils **6** aus **Figur 2** einen Kopfabschnitt **30** mit Greifbacke **31,** einen Gelenkabschnitt **32** und einen Griffabschnitt **33** auf. Wenn der zweite Greifarm **29** in Pfeilrichtung bewegt wird, öffnet sich der Greifmechanismus. Ebenso wie bei dem Aufsatzteil **6** kann der Greifmechanismus des Aufsatzteils **25** in einem unbetätigten Zustand durch beispielsweise ein federndes Element (nicht dargestellt) im Gelenkabschnitt **32** geschlossen gehalten werden. Somit kann ein Tupfer von dem Greifmechanismus selbständig fixiert gehalten werden.

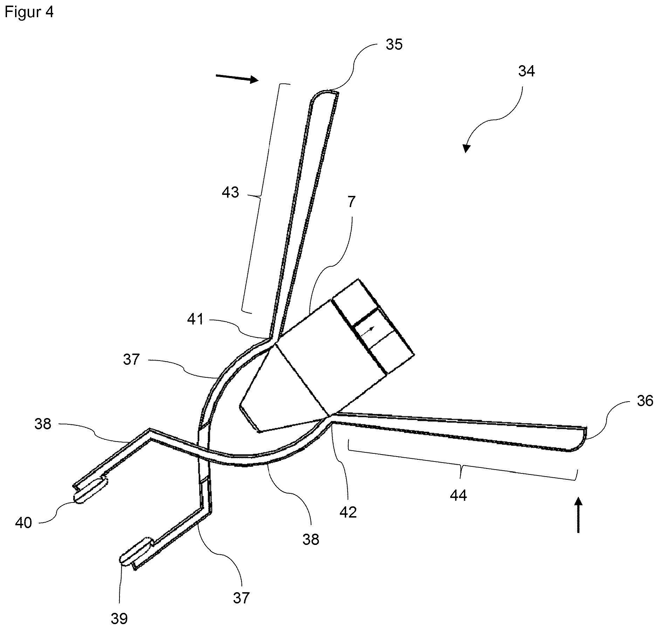

**Figur 4** zeigt ein Aufsatzteil **34** des Applikators in einer dritten Ausführungsform. Der Hohlkörper **7** ist wie bei den vorangehend beschriebenen Varianten ausgestaltet. Auch die beiden Greifarme **35, 36** weisen grundsätzlich dieselben Strukturelemente wie bei Aufsatzteil **6** auf, nämlich die Kopfabschnitte **37, 38** mit den Greifbacken **39, 40,** die Gelenkabschnitte **41, 42** sowie die Griffabschnitte **43, 44.** Allerdings überkreuzen sich die Kopfabschnitte **37, 38** anstatt entgegengesetzt bogenförmig zu verlaufen. Der Greifmechanismus ist in **Figur 4** in geöffneter Position gezeigt. Diese Position kann durch die im Zusammenhang mit **Figur 2** bereits erläuterten federnden Elemente als eine Ausgangsstellung des Greifmechanismus im unbetätigten Zustand automatisch vorliegen. Erst wenn die Greifarme **35, 36** in Pfeilrichtung bewegt werden, schließt sich der Greifmechanismus und ein Tupfer kann ergriffen und gehalten werden. Zwar kann mit einem solchen Greifmechanismus der Tupfer nicht selbständig fixiert gehalten werden, da sich der Greifmechanismus beim Loslassen der Griffabschnitte **43, 44** automatisch öffnet. Der Tupfer kann jedoch mit hoher Kraft gehalten werden, weil die manuell erzeugten Kräfte auf die Griffabschnitte **43, 44** zum Schließen des Greifmechanismus höher sein können als die von den federnden Elementen erzeugten Kräfte, welche die Griffabschnitte **23, 24** von dem Hohlkörper **7** weg drücken und den Greifmechanismus von Aufsatzteil **6** schließen. Der Vergleich der Aufsatzteile aus **Figur 2** **und** **4** macht jedenfalls deutlich, dass ein veränderter Verlauf der Kopfabschnitte bei ansonsten gleichem Aufbau zu einer Umkehr der Wirkweise des Greifmechanismus führen kann. So ist der Greifmechanismus mit den entgegengesetzt bogenförmigen Kopfabschnitten **17, 18** im unbetätigten Zustand geschlossen und öffnet sich beim Bewegen der Griffabschnitte **23, 24** gemäß **Figur 2c****,** wohingegen der Greifmechanismus mit den sich überkreuzenden Kopfabschnitten **37, 38** im unbetätigten Zustand offen ist und sich beim Bewegen der Griffabschnitte **43, 44** in Pfeilrichtung gemäß **Figur 4** schließt.

Ähnlich wie bei dem Aufsatzteil **25** aus **Figur 3** ist es prinzipiell auch hier möglich, den Kopfabschnitt **37** feststehend an dem Hohlkörper **7** auszubilden. Der Gelenkabschnitt **41** und der Griffabschnitt **43** fällt dann weg, was den Greifmechanismus vereinfacht. Dies geht jedoch ebenso wie bei Aufsatzteil **25** mit einer geringeren Beweglichkeit des Greifmechanismus einher.

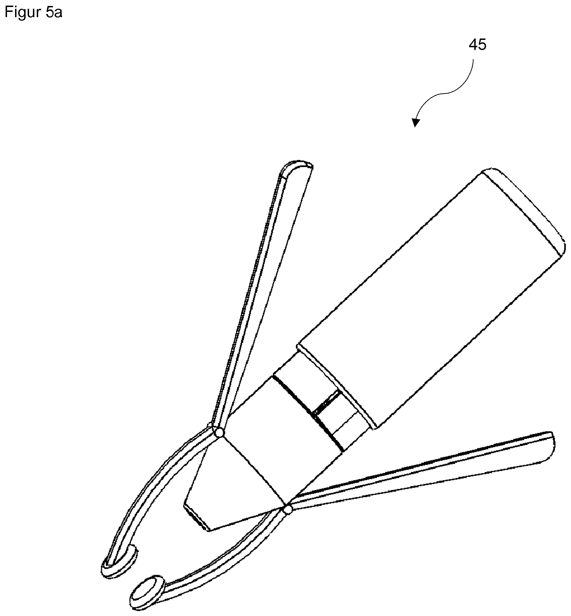

**Figur 5a** zeigt einen Applikator **45** in einer ersten Ausführungsform. Der Applikator **45** wird erhalten, indem das Aufsatzteil **6** aus **Figur 2** an den Behälter **1** aus **Figur 1** geschraubt wird. Der mit **Figur 5a** veranschaulichte Zustand des Applikators **45** wird vorliegend auch als erste Anordnung des Applikators **45** bezeichnet.

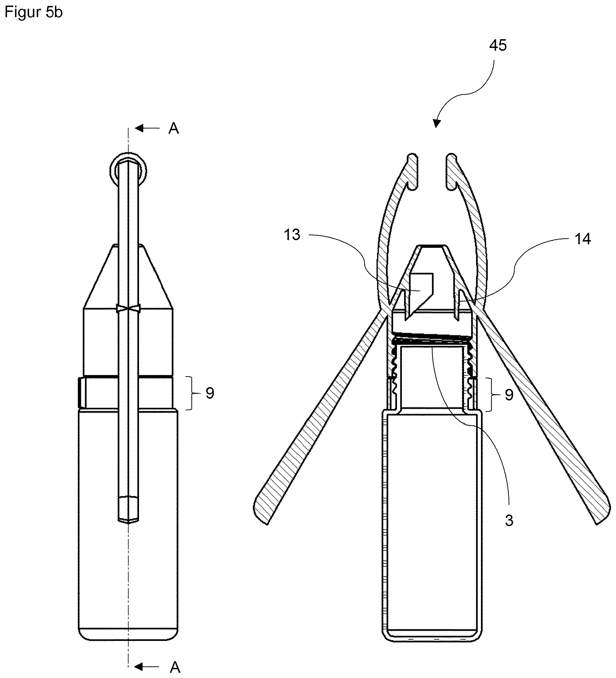

Die **Figuren 5b bis 5d** zeigen Schnittansichten des Applikators **45** aus **Figur 5a** (Schnitt entlang der Linie A-A, siehe **Figur 5b****)** und verdeutlichen die Wirkweise des Abreißringes **9** als Abstandshalter. So lässt der Abreißring **9** das Aufsatzteil **6** bereits an die Oberseite des Behälter **1** anstoßen, wenn der Membranverschluss **3** und die beiden Dorne **13, 14** noch voneinander beabstandet sind. In der Folge können die Dorne **13, 14** den Membranverschluss **3** nicht öffnen. Der Abreißring **9** verhindert also letztendlich das vollständige, zur Öffnung des Membranverschlusses **3** erforderliche Verschrauben von Aufsatzteil **6** und Behälter **1.**

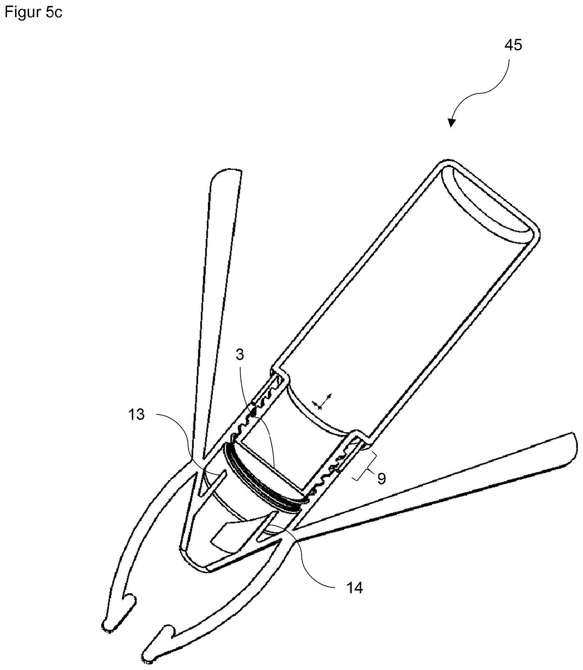

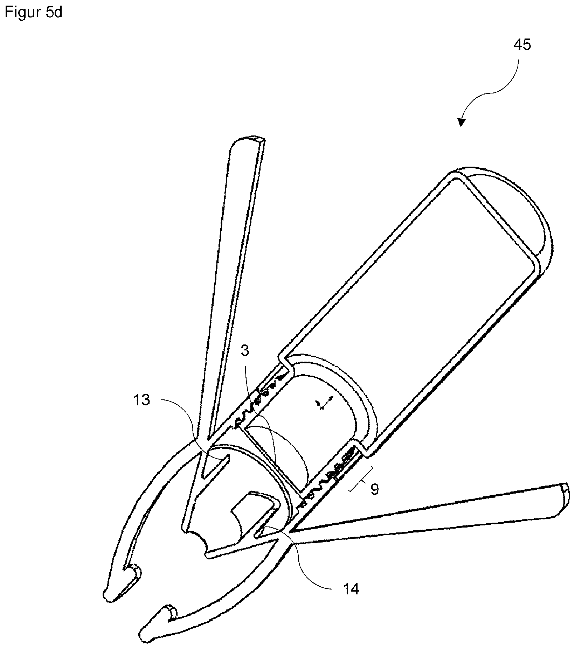

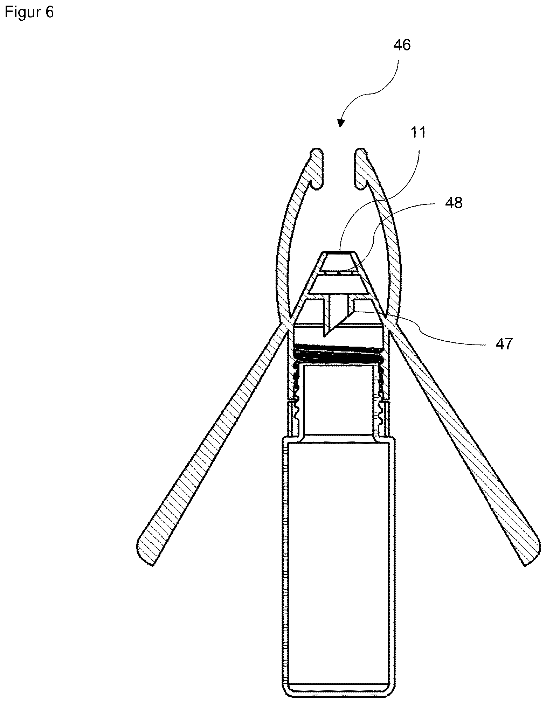

**Figur 6** zeigt einen Applikator **46** in einer zweiten Ausführungsform (Schnittansicht wie in **Figur 5b****).** Im Gegensatz zum Applikator **45** aus **Figur 5** ist hier nur ein Dorn **47** vorhanden, welcher rohrartig ausgestaltet und im Hinblick auf den Querschnitt des Aufsatzteils beziehungsweise des Hohlkörpers zentral angeordnet ist. Außerdem verfügt der Applikator **46** über ein Flatterventil **48.** Dieses ist zwischen dem rohrartigen Dorn **47** und dem zweiten Auslass **11** des Aufsatzteils angeordnet. Das Flatterventil **48** öffnet sich erst, wenn der Behälter zusammengedrückt wird.

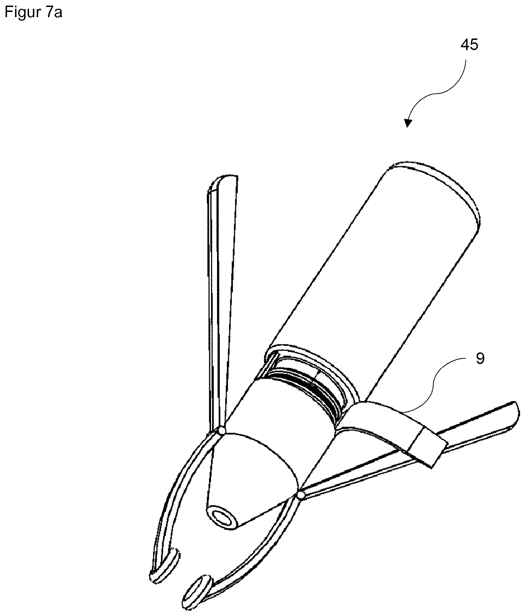

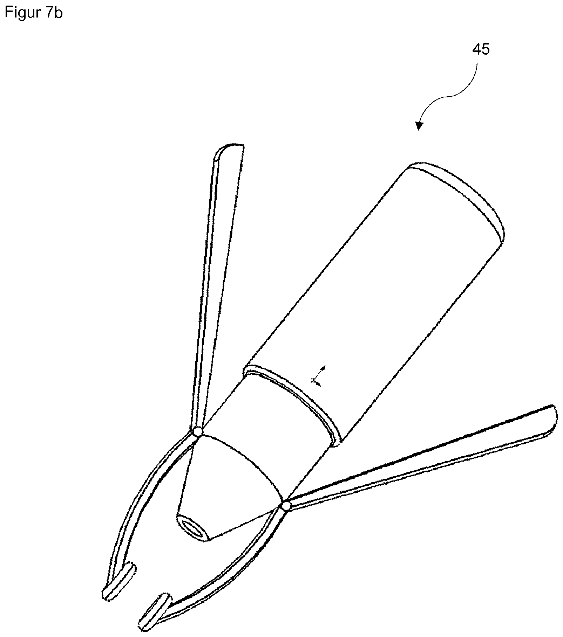

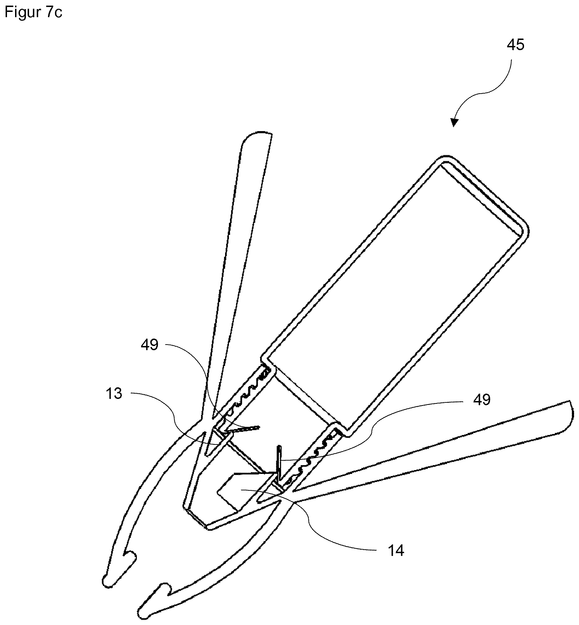

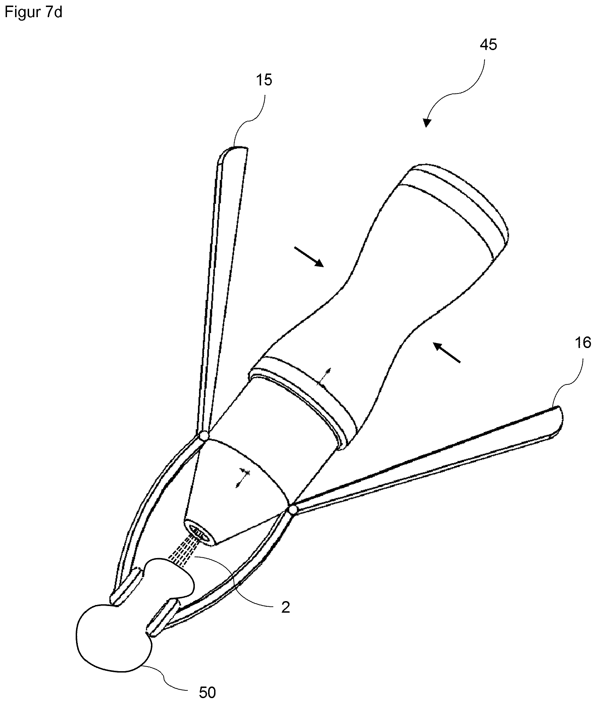

Die **Figuren 7a bis 7d** zeigen beispielhaft die Funktionsweise des Applikators **45.** **Figur 7a** veranschaulicht, wie der Abreißring **9** von dem Applikator **45** entfernt wird. Nach der vollständigen Entfernung des Abreißringes **9** ist das Aufsatzteil **6** kürzer und stößt nicht mehr an die Oberseite des Behälters **1** an. Das Aufsatzteil **6** kann dann weiter beziehungsweise vollständig auf den Behälter **1** geschraubt werden. Wird nun das verkürzte Aufsatzteil **6** bis zum Anschlag auf den Behälter **1** geschraubt **(****Figur 7b** **und** **7c****),** nähern sich die Dorne **13, 14** dem Membranverschluss **3** automatisch so weit an, dass sie diesen durchstoßen und öffnen können. Der geöffnete Membranverschluss ist in der Schnittansicht der **Figur 7c** schematisch eingezeichnet und mit dem Bezugszeichen **49** versehen. Der in **Figur 7b** **und** **7c** veranschaulichte Zustand des Applikators **45** wird vorliegend auch als zweite Anordnung des Applikators **45** bezeichnet. In **Figur 7d** ist schließlich gezeigt, wie mit dem geöffneten Applikator **45** die Flüssigkeit **2,** typischerweise ein Antiseptikum, auf einen von dem Greifmechanismus **15, 16** gehaltenen Tupfer **50** aufgetragen werden kann. Hierfür wird der geöffnete Applikator **45** mit dem Tupfer **50** nach unten gehalten (also so ausgerichtet, dass die Flüssigkeit **2** der Schwerkraft folgend aus dem Applikator **45** austreten und auf den Tupfer **50** fallen kann) und der Behälter **1** unterstützend zusammengedrückt. Mit dem feuchten, von dem Applikator **45** gehaltenen Tupfer **50** kann dann eine Hautoberfläche abgewischt und desinfiziert werden. Damit die Flüssigkeit **2** nicht vorzeitig aus dem Applikator **45** austritt, zum Beispiel beim Ergreifen des Tupfers **50,** kann ein Flatterventil (nicht dargestellt) im Aufsatzteil **6** des Applikators **45** vorgesehen sein. Das Flatterventil verhindert einen Flüssigkeitsaustritt, bis der Behälter **1** zusammengedrückt wird.

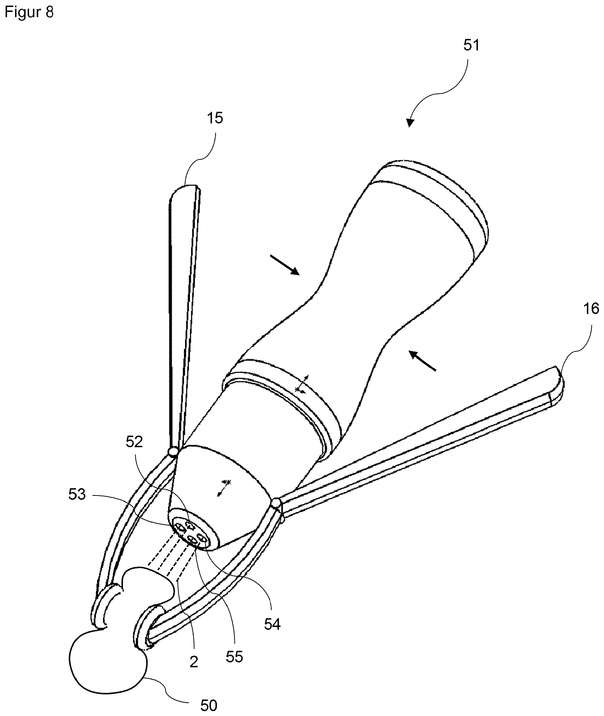

**Figur 8** zeigt einen Applikator **51,** mit dem die Flüssigkeit **2** wie bei **Figur 7d** auf den Tupfer **50** aufgetragen wird. Der Applikator **51** unterscheidet sich von dem Applikator **45** dadurch, dass der zweite Auslass mehrere Öffnungen **52, 53, 54, 55** umfasst. Die Anzahl der Öffnungen (in **Figur 8** sind vier Öffnungen eingezeichnet) ist rein beispielhaft zu verstehen. Insbesondere bilden die mehreren Öffnungen **52, 53, 54, 55** eine siebartige Anordnung aus. Dadurch kann die Flüssigkeit **2** wie in **Figur 8** angedeutet besser verteilt und besser dosiert auf den Tupfer **50** aufgetragen werden.

## Patentansprüche

1. Medizinischer Applikator (45, 46, 51) zum Auftragen einer Flüssigkeit (2), insbesondere eines Antiseptikums, auf einen Tupfer (50), umfassend
- einen flexiblen Behälter (1) mit der Flüssigkeit (2) und mit einem einen Membranverschluss (3) aufweisenden ersten Auslass (4),
- ein Aufsatzteil (6, 25, 34) für den Behälter (1), umfassend
- einen zweiten Auslass (11),
- mindestens ein Perforationselement (13, 14, 47) zum Durchstoßen und Öffnen des Membranverschlusses,
- einen entfernbaren Abstandshalter (9) zum Beabstanden von Membranverschluss und Perforationselement, und
- einen Greifmechanismus (15, 16, 26, 29, 35, 36) zum Ergreifen und Halten des Tupfers,
wobei das Aufsatzteil (6, 25, 34) an dem Behälter (1) befestigt ist und die Flüssigkeit (2) bei geöffnetem Membranverschluss (3) von dem ersten Auslass (4) zu dem zweiten Auslass (11) fließen kann, und
wobei der Applikator von einer ersten Anordnung in eine zweite Anordnung überführt werden kann, indem der Abstandshalter (9) entfernt und das Aufsatzteil (6, 25, 34) in Richtung Behälter (1) bewegt, insbesondere geschoben, gedreht oder geschraubt, werden kann, so dass das Perforationselement (13, 14, 47) den Membranverschluss (3) durchstoßen und öffnen kann,
**dadurch gekennzeichnet, dass** der Greifmechanismus manuell und zangenartig ausgestaltet ist und einen ersten Greifarm (15, 26, 35) und einen zweiten Greifarm (16, 29, 36) umfasst.

2. Medizinischer Applikator (45, 46, 51) nach Anspruch 1, wobei der Behälter (1) im Wesentlichen zylinderförmig ist.

3. Medizinischer Applikator (45, 46, 51) nach Anspruch 1 oder 2, wobei das den Behälter (1) bildende Material einen Kunststoff umfasst.

4. Medizinischer Applikator (45, 46, 51) nach einem der vorangehenden Ansprüche, wobei der zweite Auslass (11) eine einzige Öffnung (12) aufweist oder mehrere Öffnungen (52, 53, 54, 55) umfasst.

5. Medizinischer Applikator (45, 46, 51) nach einem der vorangehenden Ansprüche, wobei das Aufsatzteil (6, 25, 34) einen Hohlkörper (7) mit einem ersten offenen Ende (8) und einem zweiten offenen Ende (10) umfasst, wobei
- das erste offene Ende (8) zur Befestigung des Aufsatzteils (6, 25, 34) an dem Behälter (1) vorgesehen ist und den ersten Auslass (4) aufnehmen kann,
- das zweite offene Ende (10) den zweiten Auslass (11) bildet und dem ersten offenen Ende (8) vorzugsweise gegenüberliegend angeordnet ist,
- das Perforationselement (13, 14, 47) in dem Hohlkörper (7) angeordnet ist,
- der Abstandshalter (9) an dem ersten offenen Ende (8) angeordnet ist, und
- der erste Greifarm (15, 26, 35) und der zweite Greifarm (16, 29, 36) an dem Hohlkörper (7), vorzugsweise an dem Hohlkörper (7) an zwei sich im Wesentlichen gegenüberliegenden Positionen, befestigt sind.

6. Medizinischer Applikator (45, 46, 51) nach Anspruch 5, wobei der Hohlkörper (7) im Wesentlichen die Form eines Zylinders oder eines Kegelstumpfes aufweist, wobei
- eine Grundfläche des Zylinders oder des Kegelstumpfes das erste offene Ende (8) umfasst,
- eine Deckfläche des Zylinders oder des Kegelstumpfes das zweite offene Ende (10) umfasst, und
- der erste Greifarm (15, 26, 35) und der zweite Greifarm (16, 29, 36) an einer Mantelfläche des Zylinders oder des Kegelstumpfes befestigt sind.

7. Medizinischer Applikator (45, 46, 51) nach einem der vorangehenden Ansprüche, wobei
- sowohl der erste als auch der zweite Greifarm (15, 16, 35, 36) einen Kopfabschnitt (17, 18, 37, 38), einen Gelenkabschnitt (21, 22, 41, 42) und einen Griffabschnitt (23, 24, 43, 44) umfasst, oder
- der erste Greifarm (26) einen Kopfabschnitt (27) umfasst und feststehend mit dem Aufsatzteil (25), insbesondere feststehend mit dem Hohlkörper (7), verbunden ist, wobei der erste Greifarm (26) an der Verbindungsstelle mit dem Aufsatzteil (25), insbesondere an der Verbindungsstelle mit dem Hohlkörper (7), endet, und der zweite Greifarm (29) einen Kopfabschnitt (30), einen Gelenkabschnitt (32) und einen Griffabschnitt (33) umfasst.

8. Medizinischer Applikator (45, 46, 51) nach einem der vorangehenden Ansprüche, wobei der Greifmechanismus (15, 16, 26, 29) derart ausgestaltet ist, dass er in einem unbetätigten Zustand im Wesentlichen geschlossen ist und bei Betätigung geöffnet werden kann.

9. Medizinischer Applikator (45, 46, 51) nach Anspruch 8, wobei
- das Aufsatzteil (6, 25) mindestens ein federndes Element umfasst, so dass sich der Greifmechanismus (15, 16, 26, 29) selbstständig in eine Ausgangsstellung bewegen kann, wenn sich der Greifmechanismus (15, 16, 26, 29) in einem unbetätigten Zustand befindet, und
- der erste und der zweite Greifarm (15, 16, 26, 29), insbesondere der Kopfabschnitt (17, 18, 27, 30) des ersten und des zweiten Greifarms (15, 16, 26, 29), derart ausgestaltet sind, dass der Greifmechanismus (15, 16, 26, 29) in der Ausgangsstellung im Wesentlichen geschlossen ist und bei Betätigung geöffnet werden kann.

10. Medizinischer Applikator nach einem der Ansprüche 1 bis 7, wobei der Greifmechanismus (35, 36) derart ausgestaltet ist, dass er in einem unbetätigten Zustand geöffnet ist und bei Betätigung geschlossen werden kann.

11. Medizinischer Applikator nach Anspruch 10, wobei
- das Aufsatzteil (34) mindestens ein federndes Element umfasst, so dass sich der Greifmechanismus (35, 36) selbstständig in eine Ausgangsstellung bewegen kann, wenn sich der Greifmechanismus (35, 36) in einem unbetätigten Zustand befindet, und
- der erste und der zweite Greifarm (35, 36), insbesondere der Kopfabschnitt (37, 38) des ersten und des zweiten Greifarms (35, 36), derart ausgestaltet sind, dass der Greifmechanismus (35, 36) in der Ausgangsstellung geöffnet ist und bei Betätigung geschlossen werden kann.

12. Medizinischer Applikator (46) nach einem der vorangehenden Ansprüche, wobei der Behälter oder das Aufsatzteil ein Flatterventil (48) umfasst.

13. Medizinischer Applikator (45, 46, 51) nach einem der vorangehenden Ansprüche, wobei das Aufsatzteil (6, 25, 34) an dem Behälter (1) befestigt ist, indem das Aufsatzteil dem Behälter aufgesteckt, vorzugsweise einrastend aufgesteckt, ist oder indem das Aufsatzteil an dem Behälter angeschraubt ist.

14. Medizinischer Applikator (45, 46, 51) nach einem der vorangehenden Ansprüche, wobei das mindestens eine Perforationselement ein Dorn (13, 14, 47) ist, und wobei vorzugsweise
- das Aufsatzteil einen einzigen rohrartig ausgestalteten Dorn (47) besitzt, wobei der Dorn (47) zentral in dem Aufsatzteil angeordnet ist, oder
- das Aufsatzteil einen einzigen oder zwei flächig ausgestaltete Dorne (13, 14) besitzt, wobei die Dorne (13, 14) so in dem Aufsatzteil angeordnet sind, dass der Membranverschluss (3) in einem peripheren Bereich durchstoßen werden kann.

15. Medizinischer Applikator (45, 46, 51) nach einem der vorangehenden Ansprüche, wobei der Abstandshalter ein ringartiges Abreißelement (9), insbesondere ein Abreißring (9), ist.

16. Kit, umfassend
- einen sterilen medizinischen Applikator (45, 46, 51) nach einem der vorangehenden Ansprüche,
- optional einen oder mehrere sterile Tupfer (50), und
- optional eine Gebrauchsanweisung.

17. Verfahren zum Auftragen einer Flüssigkeit (2), insbesondere eines Antiseptikums, auf einen Tupfer (50), umfassend die Schritte:
i. Bereitstellen eines medizinischen Applikators (45) nach einem der Ansprüche 1 bis 15,
ii. Öffnen des Membranverschlusses (3) des Behälters (1), indem der Abstandshalter (9) von dem Applikator (45) entfernt und das Aufsatzteil (6) in Richtung Behälter (1) bewegt, insbesondere geschoben, gedreht oder geschraubt, wird,
iii. Bereitstellen des Tupfers (50),
iv. Ergreifen und Halten des Tupfers (50) mit dem Greifmechanismus (15, 16),
v. optional Ausrichten des Applikators (45) mit dem Tupfer (50) in eine Position, in welcher die Flüssigkeit (2) der Schwerkraft folgend aus dem Behälter (1) auf den Tupfer (50) aufgetragen werden kann,
vi. Auftragen eines Teils der in dem Behälter (1) enthaltenen Flüssigkeit (2) oder der gesamten in dem Behälter (1) enthaltenen Flüssigkeit (2) auf den Tupfer (50), indem der Behälter (1) einmal oder mehrmals zusammengedrückt wird.

## Claims

1. Medical applicator (45, 46, 51) for applying a liquid (2), in particular an antiseptic, to a swab (50), comprising
- a flexible container (1) with the liquid (2) and with a first outlet (4) having a membrane closure (3),
- an attachment part (6, 25, 34) for the container (1), comprising
- a second outlet (11),
- at least one perforation element (13, 14, 47) for piercing and opening the membrane closure,
- a removable spacer (9) for spacing apart membrane closure and perforation element, and
- a gripping mechanism (15, 16, 26, 29, 35, 36) for gripping and holding the swab,
wherein the attachment part (6, 25, 34) is fastened to the container (1), and the liquid (2) is able to flow from the first outlet (4) to the second outlet (11) when the membrane closure (3) is opened, and wherein the applicator can be transferred from a first arrangement to a second arrangement by means of the spacer (9) being removed and the attachment part (6, 25, 34) being moved, in particular pushed, rotated or screwed, in the direction of the container (1) such that the perforation element (13, 14, 47) is able to pierce and open the membrane closure (3),
**characterized in that** the gripping mechanism is a manual gripping mechanism designed like pliers and comprises a first gripping arm (15, 26, 35) and a second gripping arm (16, 29, 36).

2. Medical applicator (45, 46, 51) according to Claim 1, wherein the container (1) is substantially cylindrical.

3. Medical applicator (45, 46, 51) according to Claim 1 or 2, wherein the material forming the container (1) comprises a plastic.

4. Medical applicator (45, 46, 51) according to one of the preceding claims, wherein the second outlet (11) has a single opening (12) or comprises a plurality of openings (52, 53, 54, 55).

5. Medical applicator (45, 46, 51) according to one of the preceding claims, wherein the attachment part (6, 25, 34) comprises a hollow body (7) with a first open end (8) and a second open end (10), wherein
- the first open end (8) is provided for fastening the attachment part (6, 25, 34) to the container (1) and is able to receive the first outlet (4),
- the second open end (10) forms the second outlet (11) and is arranged preferably lying opposite the first open end (8),
- the perforation element (13, 14, 47) is arranged in the hollow body (7),
- the spacer (9) is arranged at the first open end (8), and
- the first gripping arm (15, 26, 35) and the second gripping arm (16, 29, 36) are fastened to the hollow body (7), preferably to the hollow body (7) at two substantially opposite positions.

6. Medical applicator (45, 46, 51) according to Claim 5, wherein the hollow body (7) has substantially the shape of a cylinder or of a truncated cone, wherein
- a base of the cylinder or of the truncated cone comprises the first open end (8),
- a top surface of the cylinder or of the truncated cone comprises the second open end (10), and
- the first gripping arm (15, 26, 35) and the second gripping arm (16, 29, 36) are fastened to a lateral surface of the cylinder or of the truncated cone.

7. Medical applicator (45, 46, 51) according to one of the preceding claims, wherein
- both the first and the second gripping arm (15, 16, 35, 36) have a head portion (17, 18, 37, 38), a hinge portion (21, 22, 41, 42) and a handle portion (23, 24, 43, 44), or
- the first gripping arm (26) comprises a head portion (27) and is fixedly connected to the attachment part (25), in particular fixedly connected to the hollow body (7), wherein the first gripping arm (26) ends at the connection point to the attachment part (25), in particular at the connection point to the hollow body (7), and the second gripping arm (29) comprises a head portion (30), a hinge portion (32) and a handle portion (33).

8. Medical applicator (45, 46, 51) according to one of the preceding claims, wherein the gripping mechanism (15, 16, 26, 29) is configured in such a way that it is substantially closed in an unactuated state and is able to be opened upon actuation.

9. Medical applicator (45, 46, 51) according to Claim 8, wherein
- the attachment part (6, 25) comprises at least one resilient element, such that the gripping mechanism (15, 16, 26, 29) is able to move independently to a starting position when the gripping mechanism (15, 16, 26, 29) is in an unactuated state, and
- the first and the second gripping arm (15, 16, 26, 29), in particular the head portion (17, 18, 27, 30) of the first and the second gripping arm (15, 16, 26, 29), are configured in such a way that the gripping mechanism (15, 16, 26, 29) is substantially closed in the starting position and is able to be opened upon actuation.

10. Medical applicator according to one of Claims 1 to 7, wherein the gripping mechanism (35, 36) is configured in such a way that it is open in an unactuated state and is able to be closed upon actuation.

11. Medical applicator according to Claim 10, wherein
- the attachment part (34) comprises at least one resilient element, such that the gripping mechanism (35, 36) is able to move independently to a starting position when the gripping mechanism (35, 36) is in an unactuated state, and
- the first and the second gripping arm (35, 36), in particular the head portion (37, 38) of the first and the second gripping arm (35, 36), are configured in such a way that the gripping mechanism (35, 36) is open in the starting position and is able to be closed upon actuation.

12. Medical applicator (46) according to one of the preceding claims, wherein the container or the attachment part comprises a flutter valve (48).

13. Medical applicator (45, 46, 51) according to one of the preceding claims, wherein the attachment part (6, 25, 34) is fastened to the container (1) by means of the attachment part being plugged onto the container, preferably plugged on with a latching action, or by means of the attachment part being screwed onto the container.

14. Medical applicator (45, 46, 51) according to one of the preceding claims, wherein the at least one perforation element is a mandrel (13, 14, 47), and wherein preferably
- the attachment part has a single tubular mandrel (47), wherein the mandrel (47) is arranged centrally in the attachment part, or
- the attachment part has a single or two planar mandrels (13, 14), wherein the mandrels (13, 14) are arranged in the attachment part such that the membrane closure (3) is able to be pierced in a peripheral region.

15. Medical applicator (45, 46, 51) according to one of the preceding claims, wherein the spacer is a ringlike tear-off element (9), in particular a tear-off ring (9).

16. Kit comprising
- a sterile medical applicator (45, 46, 51) according to one of the preceding claims,
- optionally one or more sterile swabs (50), and
- optionally an instruction notice.

17. Method for applying a liquid (2), in particular an antiseptic, to a swab (50), comprising the steps of:
i. making available a medical applicator (45) according to one of Claims 1 to 15,
ii. opening the membrane closure (3) of the container (1) by means of the spacer (9) being removed from the applicator (45) and by means of the attachment part (6) being moved, in particular pushed, rotated or screwed, in the direction of the container (1),
iii. making available the swab (50),
iv. gripping and holding the swab (50) with the gripping mechanism (15, 16),
v. optionally orienting the applicator (45) with the swab (50) to a position in which the liquid (2) is able to be applied by gravity from the container (1) to the swab (50),
vi. applying some of the liquid (2) contained in the container (1), or all of the liquid (2) contained in the container (1), to the swab (50) by pressing the container (1) together once or several times.

## Revendications

1. Applicateur médical (45, 46, 51) destiné à appliquer un liquide (2), notamment un antiseptique, sur un tampon (50), ledit applicateur comprenant
- un récipient souple (1) comportant le liquide (2) et pourvu d'une première sortie (4) comportant une fermeture à membrane (3),
- une pièce de fixation (6, 25, 34) destinée au récipient (1) et comprenant
- une deuxième sortie (11),
- au moins un élément de perforation (13, 14, 47) destiné à percer et ouvrir la fermeture à membrane,
- une élément d'espacement amovible (9) destiné à espacer la fermeture à membrane et l'élément de perforation, et
- un mécanisme de préhension (15, 16, 26, 29, 35, 36) destiné à saisir et maintenir le tampon,
la pièce de fixation (6, 25, 34) étant fixée au récipient (1) et le liquide (2) pouvant s'écouler de la première sortie (4) à la deuxième sortie (11) lorsque la fermeture à membrane (3) est ouverte, et l'applicateur pouvant être transféré d'un premier ensemble à un deuxième ensemble par retrait de l'élément d'espacement (9) et déplacement de la pièce de fixation (6, 25, 34), notamment par coulissement, rotation ou vissage, en direction du récipient (1), de sorte que l'élément de perforation (13, 14, 47) puisse percer et ouvrir la fermeture à membrane (3),
**caractérisé en ce que** le mécanisme de préhension est conçu pour être manuel et semblable à une pince et comprend un premier bras de préhension (15, 26, 35) et un deuxième bras de préhension (16, 29, 36).

2. Applicateur médical (45, 46, 51) selon la revendication 1, le récipient (1) étant sensiblement cylindrique.

3. Applicateur médical (45, 46, 51) selon la revendication 1 ou 2, le matériau qui forme le récipient (1) comprenant une matière synthétique.

4. Applicateur médical (45, 46, 51) selon l'une des revendications précédentes, la deuxième sortie (11) comporte une seule ouverture (12) ou comprend une pluralité d'ouvertures (52, 53, 54, 55).

5. Applicateur médical (45, 46, 51) selon l'une des revendications précédentes, la pièce de fixation (6, 25, 34) comprenant un corps creux (7) pourvu d'une première extrémité ouverte (8) et d'une deuxième extrémité ouverte (10),
- la première extrémité ouverte (8) étant prévue pour fixer la pièce de fixation (6, 25, 34) au récipient (1) et pouvant recevoir la première sortie (4),
- la deuxième extrémité ouverte (10) formant la deuxième sortie (11) et étant de préférence disposée en regard de la première extrémité ouverte (8),
- l'élément de perforation (13, 14, 47) étant disposé dans le corps creux (7),
- l'élément d'espacement (9) étant disposé à la première extrémité ouverte (8), et
- le premier bras de préhension (15, 26, 35) et le deuxième bras de préhension (16, 29, 36) étant fixés au corps creux (7), de préférence au corps creux (7) en deux positions sensiblement opposées.

6. Applicateur médical (45, 46, 51) selon la revendication 5, le corps creux (7) ayant sensiblement la forme d'un cylindre ou d'un cône tronqué,
- une surface de base du cylindre ou du cône tronqué comprenant la première extrémité ouverte (8),
- une surface supérieure du cylindre ou du cône tronqué comprenant la deuxième extrémité ouverte (10), et
- le premier bras de préhension (15, 26, 35) et le deuxième bras de préhension (16, 29, 36) étant fixés sur une surface latérale du cylindre ou du cône tronqué.

7. Applicateur médical (45, 46, 51) selon l'une des revendications précédentes,
- le premier et le deuxième bras de préhension (15, 16, 35, 36) comprenant une portion de tête (17, 18, 37, 38), une portion d'articulation (21, 22, 41, 42) et une portion de préhension (23, 24, 43, 44), ou
- le premier bras de préhension (26) comprenant une portion de tête (27) et étant relié de manière fixe à la partie de fixation (25), en particulier de manière fixe au corps creux (7), le premier bras de préhension (26) se terminant au point de liaison avec la partie de fixation (25), en particulier au point de liaison avec le corps creux (7), et le deuxième bras de préhension (29) comprenant une portion de tête (30), une portion d'articulation (32) et une portion de préhension (33).

8. Applicateur médical (45, 46, 51) selon l'une des revendications précédentes, le mécanisme de préhension (15, 16, 26, 29) étant conçu de manière à être sensiblement fermé lorsqu'il n'est pas actionné et de manière à pouvoir être ouvert lorsqu'il est actionné.

9. Applicateur médical (45, 46, 51) selon la revendication 8,
- la pièce de fixation (6, 25) comprenant au moins un élément à ressort de sorte que le mécanisme de préhension (15, 16, 26, 29) puisse se déplacer indépendamment dans une position initiale lorsque le mécanisme de préhension (15, 16, 26, 29) n'est pas actionné, et
- le premier et le deuxième bras de préhension (15, 16, 26, 29), en particulier la portion de tête (17, 18, 27, 30) du premier et du deuxième bras de préhension (15, 16, 26, 29) étant conçus de telle sorte que le mécanisme de préhension (15, 16, 26, 29) soit sensiblement fermé dans la position initiale et puisse être ouvert lorsqu'il est actionné.

10. Applicateur médical selon l'une des revendications 1 à 7, le mécanisme de préhension (35, 36) étant conçu de manière à être ouvert lorsqu'il n'est pas actionné et de manière à être fermé lorsqu'il est actionné.

11. Applicateur médical selon la revendication 10,
- la pièce de fixation (34) comprenant au moins un élément à ressort de sorte que le mécanisme de préhension (35, 36) puisse se déplacer indépendamment dans une position initiale lorsque le mécanisme de préhension (35, 36) n'est pas actionné, et
- le premier et le deuxième bras de préhension (35, 36), en particulier la portion de tête (37, 38) du premier et du deuxième bras de préhension (35, 36), sont conçus de manière à ce que le mécanisme de préhension (35, 36) soit ouvert en position initiale et puisse être fermé lorsqu'il est actionné.

12. Applicateur médical (46) selon l'une des revendications précédentes, le récipient ou la pièce de fixation comprenant une soupape à flottement (48).

13. Applicateur médical (45, 46, 51) selon l'une des revendications précédentes, la pièce de fixation (6, 25, 34) étant fixée au récipient (1) par emboîtement de la pièce de fixation sur le récipient, de préférence par encliquetage, ou la pièce de fixation étant vissée au récipient.

14. Applicateur médical (45, 46, 51) selon l'une des revendications précédentes, l'au moins un élément de perforation étant un mandrin (13, 14, 47), et de préférence
- la pièce de fixation comportant un seul mandrin tubulaire (47), le mandrin (47) étant disposé au centre dans la pièce de fixation, ou
- la pièce de fixation comportant un seul mandrin ou deux mandrins (13, 14) sensiblement bidimensionnels, les mandrins (13, 14) étant disposés dans la pièce de fixation de manière à ce que la fermeture à membrane (3) puisse être percée dans une zone périphérique.

15. Applicateur médical (45, 46, 51) selon l'une des revendications précédentes, l'élément d'espacement étant un élément déchirable (9) en forme d'anneau, notamment un anneau déchirable (9).

16. Kit comprenant
- un applicateur médical stérile (45, 46, 51) selon l'une des revendications précédentes,
- éventuellement un ou plusieurs tampons stériles (50), et
- éventuellement un mode d'emploi.

17. Procédé d'application d'un liquide (2), notamment d'un antiseptique, sur un tampon (50), ledit procédé comprenant les étapes suivantes :
i. fournir un applicateur médical (45) selon l'une des revendications 1 à 15,
ii. ouverture la fermeture à membrane (3) du récipient (1) par retrait de l'élément d'espacement (9) de l'applicateur (45) et par déplacement de la pièce de fixation (6) en direction du récipient (1), notamment par coulissement, rotation ou vissage,
iii. fournir le tampon (50),
iv. saisir et maintenir le tampon (50) avec le mécanisme de préhension (15, 16),
v. éventuellement orienter l'applicateur (45) pourvu du tampon (50) dans une position dans laquelle le liquide (2) peut être appliqué du récipient (1) sur le tampon (50) suivant la force de gravité,
vi. appliquer une partie du liquide (2) contenu dans le récipient (1) ou tout le liquide (2) contenu dans le récipient (1) sur le tampon (50) par compression du récipient (1) une ou plusieurs fois.
